# EUROPÄISCHE PATENTSCHRIFT

(11) **EP 2 494 944 B1**
(45) Veröffentlichungstag und Bekanntmachung des Hinweises auf die Patenterteilung: **02.04.2025**
(21) Anmeldenummer: 12157361.2
(22) Anmeldetag: 28.02.2012
(51) Int. Cl.: F24F 7/013, F24F 12/00, F24F 13/18, E06B 9/52, A61F 5/441, B01D 53/04, E06B 7/10, E06B 7/02

(54) **FILTERELEMENT ZUR REINIGUNG VON AUS EINEM STOMABEUTEL AUSTRETENDEN GASEN**
FILTER ELEMENT FOR CLEANING GASES EMITTED FROM A COLOSTOMY BAG
ELÉMENT DE FILTRE DESTINÉ AU NETTOYAGE DE GAZ SORTANT D'UNE POCHE D'EXTRACTION

(30) Priorität: 01.03.2011 DE 102011004929
(43) Veröffentlichungstag der Anmeldung: 05.09.2012
(73) Patentinhaber: MANN + HUMMEL Molecular GmbH, 95502 Himmelkron (DE); For Life GmbH, 12459 Berlin (DE)
(72) Erfinder: Brie, Karl-Thomas, 10245 Berlin (DE); Ernst, Rainer, 95463 Bindlach (DE); Bilek, Thorsten, 95213 Münchberg (DE)
(74) Vertreter: Dr. Gassner & Partner mbB

(56) Entgegenhaltungen:
- EP-A2- 1 514 528
- DE-B3- 102008 044 356
- DE-U1- 29 505 030
- DE-U1- 29 620 875
- GB-A- 2 059 797

## Beschreibung

Die Erfindung betrifft ein Filterelement zur Reinigung von aus einem Stomabeutel austretenden Gasen und einen Stomabeutel.

Bei einem Stoma handelt es sich um einen durch Operation hergestellten künstlichen Darmausgang. An das Stoma wird zur Aufnahme des Verdauungsbreies ein sogenannter Stomabeutel angeschlossen, der je nach Konsistenz und Menge des Verdauungsbreies mehrfach entleert oder als Ganzes verworfen werden kann. Ein Stomabeutel wird üblicherweise über einen Zeitraum von etwa 24 Stunden benutzt. Bei allen Stomabeuteln ist es erforderlich, ein Gasauslasssystem zur Ableitung von sich bei der Verdauung bildenden Gasen vorzusehen. Bei dem Gas Auslasssystem handelt es sich im einfachsten Fall um eine Öffnung im Stomabeutel. Um hier eine Geruchsbelästigung zu vermeiden, ist in das Gasauslasssystem üblicherweise ein Aktivkohlefilter zur Reinigung der Gase integriert. Der Stomabeutel sollte möglichst über dessen üblichen Nutzungszeitraum hinweg einsatzfähig sein und so lange sowohl einen Gasauslass als auch eine Geruchsabsorption ermöglichen.

Filterelemente zur Reinigung von aus einem Stomabeutel austretenden Gasen sind beispielsweise aus der DE 10 2008 044 356 B3 als Aktivkohlefilter, die in Stomabeuteln eingesetzt werden, bekannt. Dabei weist das Filterelement eine Einlassöffnung zum Eintritt der Gase, eine Auslassöffnung zum Austritt der Gase und eine von den Gasen auf ihrem Weg von der Einlassöffnung zur Auslassöffnung zu durchströmende Aktivkohle enthaltende Schicht auf.

Aus der GB 2 059 797 A ist ein für Stomabeutel geeigneter Gasfilter bekannt, der ein poröses Filterkissen mit gegenüberliegenden ebenen Flächen und einem Netz thermoplastischer Fasern, welches mit fein verteilter Aktivkohle klebend beschichtet ist, umfasst. Der Gasfilter weist eine heißsiegelbare gasdurchlässige Flüssigkeitsbarriere auf, welche im Wesentlichen über die gesamte Ausdehnung einer der Flächen des Filters fixiert ist.

Ein bei den bisher eingesetzten Aktivkohlefiltern auftretendes Problem besteht darin, dass die Filter einerseits vor von außen, etwa beim Duschen, eintretendem Wasser geschützt werden müssen, weil ein nasser Aktivkohlefilter keine effektive Gasreinigung mehr ermöglicht. Andererseits muss auch zuverlässig verhindert werden, dass Flüssigkeit vom Inneren des Stomabeutels nach außen tritt. Bei alledem muss zuverlässig sichergestellt werden, dass Gas aus dem Stomabeutel austreten kann. Ein Gasstau ist für die Patienten sehr schmerzhaft.

Im Stand der Technik wird dieses Problem dadurch gelöst, dass ein Aktivkohlefilter zum Schutz vor Kontamination von außen im Stomabeutel hinter einer außen am Stomabeutel angeordneten für Gas, nicht aber für Flüssigkeit, durchlässigen GoreTex^{®}-Membran angeordnet wird, so dass das austretende Gas nach dem Passieren des Aktivkohlefilters die GoreTex^{®}-Membran passieren muss. Derartige Systeme sind jedoch in der Herstellung wegen des Preises der GoreTex^{®}-Membran und wegen des Aufwands, der mit deren Verarbeitung durch Kleben und der dazu erforderlichen Zeit verbunden ist, aufwändig und teuer.

Aus der DE 30 36 009 A1 ist eine geruchsabsorbierende, gasdurchlässige Filteranordnung für eine Sammeleinrichtung, wie einen Stomabeutel, bekannt. Dabei sind ein poröses Aktivkohle enthaltendes Filterkissen mit einem Paar aneinander gegenüberliegender, ebener Flächen und eine wärmeverklebbare, gasdurchlässige Flüssigkeitssperre, die an einer der Flächen über deren im Wesentlichen gesamte Erstreckung befestigt ist, vorgesehen. Die wärmeverklebbare, gasdurchlässige Flüssigkeitssperre kann ein Laminat sein, das eine poröse, thermoplastische Schicht umfasst, die auf eine wärmeverklebbare, faserige Schicht aufgeklebt ist. Beispielsweise kann ein Zellulosematerial verwendet werden, welches mit einer Ethyl-Vinyl-Acetat-Latex-Emulsion oberflächenbeschichtet ist und das luftdurchlässig, wasserbeständig und schweißfähig ist. Dieses Zellulosematerial kann beispielsweise mit einer GoreTex^{®}-Membran verbunden sein, um so die Sperrwirkung für Flüssigkeit bereitzustellen. Auch diese Anordnung ist teuer und aufwendig herzustellen.

Aufgabe der Erfindung ist es, ein Filterelement anzugeben, mit dem die Nachteile nach dem Stand der Technik vermieden werden. Insbesondere soll ein Filterelement bereitgestellt werden, welches günstig herzustellen ist und mit geringem Aufwand so in einen Stomabeutel integriert werden kann, dass es vor dem Eindringen von Wasser von außen geschützt ist und gleichzeitig den Austritt von Flüssigkeit aus dem Stomabeutel verhindert. Weiterhin soll ein Stomabeutel mit einem solchen Filterelement angegeben werden.

Diese Aufgabe wird durch die Merkmale der Ansprüche 1 und 12 gelöst. Zweckmäßige Ausgestaltungen ergeben sich aus den Merkmalen der Ansprüche 2 bis 11.

Nach Maßgabe der Erfindung ist ein Filterelement zur Reinigung von aus einem Stomabeutel austretenden Gasen vorgesehen, wobei das Filterelement mindestens eine Einlassöffnung zum Eintritt der Gase, einen Auslassbereich zum Austritt der Gase und eine von den Gasen auf ihrem Weg von der Einlassöffnung zum Auslassbereich zu durchströmende Aktivkohle enthaltende Schicht aufweist. Das Filterelement ist dabei streifenförmig ausgebildet, wobei ein Teil der äußeren Seitenflächen, nicht aber der Stirnflächen der Schicht von einer gas- und flüssigkeitsundurchlässigen Kunststofffolie umgeben und mit dieser Kunststofffolie verbunden ist. Der nicht von der Kunststofffolie umgebene Teil der äußeren Seitenflächen der Schicht bildet dabei den Auslassbereich, der sich längs des Filterelements erstreckt und der von einer für Wasser in flüssiger Form undurchlässigen aber für Wasserdampf und Gas durchlässigen thermisch oder mittels Ultraschall schweißbaren Membran bedeckt und mit dieser Membran verbunden ist, wobei der Schmelzpunkt der Membran unter 180°C, insbesondere unter 160°C, insbesondere unter 150°C, insbesondere unter 145°C, liegt.

Die Membran kann einstückig ausgebildet sein, d. **h.,** dass die Membran nicht, beispielsweise als Laminat, aus unterschiedlichen Schichten zusammengesetzt ist, von denen eine die Schweißbarkeit und eine andere die Undurchlässigkeit für Wasser in flüssiger Form bereitstellt. Einstückig bedeutet dabei aber nicht, dass die Membran keine Klebstoffschicht, **z. B.** aus einem Heißkleber, aufweisen kann. Die Schicht kann flach ausgebildet sein.

Der große Vorteil des erfindungsgemäßen Filterelements besteht darin, dass es sehr einfach und kostengünstig hergestellt werden kann, indem die Aktivkohle enthaltende Schicht in dem Auslassbereich mit der Membran und darum herum, mit Ausnahme der mindestens einen Einlassöffnung, mit der Kunststofffolie, beispielsweise durch Kleben, verbunden wird. Dadurch, dass das Filterelement streifenförmig ausgebildet ist, kann dazu beispielsweise ein erstes Band aus einem die Schicht bildenden Material von einer Rolle kontinuierlich abgerollt und auf einem Teil einer Seitenfläche kontinuierlich mit einem zweiten Band aus dem die Membran bildenden Material beklebt und auf dem Rest dieser Seitenfläche und den sonstigen Seitenflächen teilweise überlappend mit dem zweiten Band kontinuierlich mit der Kunststofffolie beklebt werden. Die erfindungsgemäßen streifenförmigen Filterelemente können von dem sich daraus ergebenden Band kontinuierlich abgeschnitten werden.

Alternativ kann ein die Schicht bildendes Material zunächst flächig ausgebildet sein und auf einer Oberfläche komplett mit der Membran beklebt werden. Das Bekleben kann mittels eines herkömmlichen Klebevlieses oder mittels eines Heißklebers erfolgen. Das Klebevlies oder der Heißkleber kann dazu auf dem die Schicht bildenden Material oder auf der Membran aufgebracht werden oder bereits zuvor darauf aufgebracht worden sein. Das mit der Membran beklebte Material kann dann in lange Streifen geschnitten werden, welche jeweils auf den nicht mit der Membran beklebten Seitenflächen und teilweise überlappend mit der Membran mit der Kunststofffolie beklebt werden. Die erfindungsgemäßen Filterelemente können dann jeweils von den daraus hervorgehenden langen Streifen abgeschnitten werden.

Weiterhin kann ein solches Filterelement sehr kostengünstig in einen Stomabeutel integriert werden. Dadurch, dass hier ein sich längs des Filterelements erstreckender und damit großflächiger Auslassbereich vorgesehen ist, muss das Filterelement nicht wie bei einem Filterelement mit punktueller Auslassöffnung aufwendig justiert werden, um die Auslassöffnung mit einer äußeren Gasauslassöffnung des Stomabeutels in Deckung zu bringen. Das erfindungsgemäße Filterelement wird hier einfach so eingesetzt, dass sich die äußere Gasauslassöffnung des Stomabeutels zumindest teilweise mit dem Auslassbereich deckt. Anschließend wird der Rand der Gasauslassöffnung mit der Membran thermisch oder mittels Ultraschall verschweißt, so dass hier eine zuverlässige Abdichtung gegenüber Wasser erreicht wird. Sofern auch die Kunststofffolie aus einem thermisch oder mittels Ultraschall schweißbaren Material besteht, kann auch ein Teil der Kunststofffolie mit dem Rand der Gasauslassöffnung des Stomabeutels verschweißt werden. Wesentlich ist dabei nur, dass eine so große Fläche der Membran innerhalb der Gasauslassöffnung angeordnet ist, dass dadurch ein sicherer Gasaustritt gewährleistet ist. Bei dem streifenförmigen Filterelement befindet sich üblicherweise je eine Einlassöffnung an jedem der Enden des Streifens. Die Enden sind üblicherweise die Schnittflächen, welche beim Abschneiden der Stücke von dem Band oder den Streifen entstehen.

GoreTex^{®} besteht aus Polytetrafluorethylen mit einem Schmelzpunkt von 327°C. Es lässt sich daher nur schlecht schweißen. Insbesondere das Verschweißen mit dem üblicherweise für die Herstellung von Stomabeuteln verwendeten Kunststoff, wie beispielsweise Polyethylen, welches bereits bei 105 bis 145°C schmilzt, ist kaum möglich. Eine Membran mit einem Schmelzpunkt unter 180°C ist daher erheblich leichter zu verarbeiten und durch Schweißen mit dem Kunststoff des Stomabeutels zu verbinden. Die Herstellung eines Stomabeutels mit darin enthaltenem Filterelement und wasserdicht mit der Membran verschweißter Gasauslassöffnung des Stomabeutels ist mit dem erfindungsgemäßen Filterelement daher erheblich einfacher und kostengünstiger zu bewerkstelligen als mit der dazu bisher üblicherweise verwendeten GoreTex^{®}-Membran, welche normalerweise mittels eines Klebstoffs mit dem Kunststoff des Stomabeutels verbunden werden muss.

Die Membran kann Polycarbonat, Polyvinylidenfluorid (PVDF) oder Acrylcopolymer, insbesondere in Form einer mikroporösen Kapillarporenmembran, umfassen oder daraus bestehen. Die Membran kann auch zu einem Vlies oder einem Gewebe verarbeitete Fasern umfassen oder aus solchen Fasern bestehen. Die Fasern können dabei thermisch verschweißt sein. Bei den Fasern kann es sich um Fasern aus Polyethylen, insbesondere Polyethylen hoher Dichte (HDPE), handeln. Kommerziell wird ein solches Material beispielsweise unter dem Handelsnamen "Tyvek" von der Firma DuPont, Wilmington, USA, vertrieben. Dabei handelt es sich um ein papierartiges Vlies aus thermisch verschweißten Fasern aus Polyethylen hoher Dichte.

Die Kunststofffolie besteht vorzugsweise aus einem thermisch oder mittels Ultraschall mit der Membran verschweißbaren Material, insbesondere Ethylenvinylacetat (EVA), Polyethylen (PE), Polypropylen (PP) oder Ethylenvinylacetat-Polyethylen Coextrudat oder Copolymer (EVA-PE). Durch ein Verschweißen der Membran mit der Kunststofffolie kann am Übergang zwischen der Membran und der Kunststofffolie zuverlässig eine gegenüber flüssigem Wasser dichte Verbindung hergestellt werden. Weiterhin ist es im Hinblick auf eine Wasserdichtheit auch unproblematisch, wenn beim Verschweißen der Gasauslassöffnung des Stomabeutels mit der Membran ein Teil der Gasauslassöffnung mit der Kunststofffolie überlappt und mit dieser verschweißt wird.

Bei einer Ausgestaltung des erfindungsgemäßen Filterelements ist der überwiegende Teil der von den äußeren Seitenflächen der Schicht gebildeten Fläche von der Kunststofffolie umgeben und damit verbunden. Die Verbindung der Kunststofffolie mit der Schicht kann ebenso wie die Verbindung der Membran mit der Schicht mittels eines thermischen oder nicht-thermischen Klebstoffs bereitgestellt werden. Der Schmelzpunkt des thermischen Klebstoffs liegt vorzugsweise unterhalb des Schmelzpunkts der Membran. Der Klebstoff kann auf der Kunststofffolie, der Membran und/oder dem die Schicht bildenden Material aufgebracht werden. Alternativ kann auch eine mit dem Klebstoff bereits beschichtete Membran und/oder Kunststofffolie und/oder ein bereits mit dem Klebstoff beschichtetes die Schicht bildendes Material eingesetzt werden. Bei dem thermischen, d. **h.** heißklebenden, Klebstoff kann es sich um ein Klebevlies oder einen flüssig oder pastös aufgetragenen Klebstoff handeln. Der Klebstoff kann aus Ethylenvinylacetat (EVA), Polyethylen (PE), Polyamid (PA), insbesondere Nylon^{®}, Polyester oder Ethylenvinylacetat-Polyethylen Coextrudat oder Copolymer (EVA-PE) bestehen.

Bei einer vorteilhaften Ausgestaltung ist das Filterelement in ein Gasauslasssystem eines Stomabeutels integriert. Dabei ist der Rand einer nach außen führenden Öffnung des Stomabeutels vollständig so mit der Membran oder der Membran und dem Kunststoff verschweißt, dass kein Wasser in flüssiger Form unter Normaldruck, d. **h.** 1,013 bar, von außen durch die Öffnung in den Stomabeutel eindringen, Gas jedoch durch die Membran aus dem Stomabeutel austreten kann. Bei dem durch ein solches Filterelement zu reinigenden Gas handelt es sich um bei der Verdauung entstehendes Gas. Durch die Reinigung mittels Aktivkohle werden aus dem Gas Bestandteile absorbiert, welche Ursache unangenehmer Gerüche sind.

Neben der Schicht kann mindestens eine weitere Schicht vorgesehen sein, die ein Feuchtigkeitsabsorptionsmittel umfasst, wobei die weitere Schicht mit der Schicht zur Aufnahme von Feuchtigkeit aus der Schicht in direktem Kontakt steht. Die weitere Schicht ist dabei so bemessen, dass sie es auch bei einer Sättigung mit Flüssigkeit noch erlaubt, dass die Gase das Filterelement von der Einlassöffnung zum Auslassbereich durchströmen, wobei die äußeren Seitenflächen der weiteren Schicht zusammen mit dem nicht den Auslassbereich bildenden Teil der äußeren Seitenflächen der Schicht von der Kunststofffolie umgeben und mit ihr verbunden sind. Die innere, der Schicht zugewandte Seitenfläche der weiteren Schicht steht dabei in direktem Kontakt mit der inneren der weiteren Schicht zugewandten Seitenfläche der Schicht. Die Schicht ist in diesem Fall teilweise indirekt, nämlich über die weitere Schicht, mit der Kunststofffolie verbunden.

Die Filterwirkung der die Aktivkohle enthaltenen Schicht kann dadurch wesentlich länger aufrechterhalten werden als ohne die Feuchtigkeit absorbierende weitere Schicht. Ein Funktionsverlust der Schicht durch Nässe wird so lange verzögert, wie die weitere Schicht die Feuchtigkeit aus der Schicht aufsaugen kann. Darüber hinaus wird die Durchlässigkeit des Filterelements für das Gas länger aufrechterhalten, als ohne die weitere Schicht. In das Filterelement eindringende kleinere Mengen an Verdauungsbrei, welche das Filterelement ansonsten zusetzen würden, werden durch den Entzug von Flüssigkeit durch die weitere Schicht soweit getrocknet und dadurch in ihrem Volumen reduziert, dass sie einen Gasdurchtritt weiterhin erlauben. Das Filterelement wird dadurch nicht so schnell verstopft. Ein Verschluss des Filterelements und ein damit verbundener Druckaufbau im Stomabeutel sowie ein dadurch erforderliches Wechseln des Stomabeutels oder zumindest des Filterelements werden weitgehend vermieden. Selbst wenn die weitere Schicht mit Flüssigkeit gesättigt ist und kein Trocknen der Schicht mehr bewirken kann, wird ein Durchtritt der Gase durch das Filterelement noch gewährleistet, auch wenn die Schicht keine geruchsabsorbierende Wirkung mehr hat.

Vorzugsweise ist die Aktivkohle auf oder in einem Trägermaterial angeordnet oder fixiert. Die Aktivkohle kann in das Trägermaterial integriert sein oder auf der Oberfläche des Trägermaterials immobilisiert sein. Das Trägermaterial kann faserförmig oder schwammförmig ausgebildet sein. Bevorzugt ist das Trägermaterial ein aus einem Kunststoff gebildeter Schaum. Bei dem Kunststoff kann es sich beispielsweise um Polyurethan handeln.

Vorzugsweise umfasst die weitere Schicht zur Aufnahme von Feuchtigkeit einen Superabsorber. Superabsorber sind beispielsweise zur Aufnahme von Feuchtigkeit in Windeln oder Inkontinenzeinlagen bekannt. Der Superabsorber kann in Form eines grobkörnigen Pulvers oder eines Granulats vorliegen, welches beispielsweise durch Einschluss in Kompartimente immobilisiert ist. Der Superabsorber steht mit der Schicht in Kontakt, so dass Feuchtigkeit, beispielsweise durch Kapillarkräfte, aus der Schicht in die weitere Schicht gelangen kann. Das getrennte Vorhandensein der Schicht und der weiteren Schicht ist vorteilhaft, weil der Superabsorber bei Aufnahme von Flüssigkeit aufquillt. Würde der Superabsorber innerhalb der die Aktivkohle enthaltenden Schicht vorliegen ohne von dieser Schicht getrennt zu sein, würde diese Schicht bei Feuchtigkeitsaufnahme aufquellen und ihre Effizienz zumindest teilweise verlieren. Im Extremfall könnte es dadurch sogar zu einem Verschluss des Filterelements kommen.

Besonders vorteilhaft ist es, wenn der Superabsorber in Form von, insbesondere zu einem Vlies verarbeiteten, Fasern vorliegt. Die Fasern können bei Flüssigkeitsaufnahme im Vlies aufquellen, ohne dass sich das vom Vlies eingenommene Gesamtvolumen stark erhöht, da durch das Aufquellen lediglich in dem Vlies enthaltene Hohlräume ausgefüllt werden. Der Superabsorber kann auch auf oder in einem, insbesondere faserförmig vorliegenden, weiteren Trägermaterial vorliegen. Vorzugsweise ist dieses Trägermaterial zu einem Vlies verarbeitet. Die prozentuale Volumenzunahme eines solchen den Superabsorber umfassenden zu einem Vlies verarbeiteten Trägermaterials bei Feuchtigkeitsaufnahme ist noch geringer als bei einem ausschließlich aus Superabsorber bestehenden Vlies, wenn das Trägermaterial selbst keine Feuchtigkeit aufnimmt oder bei Feuchtigkeitsaufnahme nicht oder zumindest nicht wesentlich aufquillt. Sowohl das Trägermaterial als auch der Superabsorber können faserförmig vorliegen und zusammen eine Vliesstruktur bilden.

Das streifenförmige Filterelement ist flach und breit ausgebildet. Die streifenförmige Ausbildung des Filterelements ist besonders vorteilhaft, weil es nur sehr gering aufträgt und ein unauffälliges Tragen des Stomabeutels unter der Kleidung ermöglicht. Weiterhin ermöglicht die Streifenform eine sehr kostengünstige Produktion durch kontinuierliche Herstellung eines langen Bands, von dem die Filterelemente jeweils abgeschnitten werden.

Bevorzugt ist die weitere Schicht dünner als die Schicht. Insbesondere ist die weitere Schicht vorzugsweise weniger als halb, vorzugsweise weniger als ein Viertel, insbesondere weniger als ein Achtel, so dick wie die Schicht. Die Dicke der weiteren Schicht wird stets so gewählt, dass die weitere Schicht durch Aufquellen bei Aufnahme derjenigen Feuchtigkeitsmenge, die sie maximal aufnehmen kann, d. h. bei Sättigung, das Filterelement nicht verschließen kann.

Bei einer Ausgestaltung des erfindungsgemäßen Filterelements steht die weitere Schicht mit der Schicht so in Kontakt, dass der Anteil einer Kontaktfläche zwischen der weiteren Schicht und der Schicht an der gesamten äußeren Oberfläche der weiteren Schicht einschließlich der Kontaktfläche in trockenem Zustand mindestens 44%, vorzugsweise mindestens 47%, beträgt. Unter der gesamten äußeren Oberfläche wird dabei nur die die weitere Schicht begrenzende Fläche und nicht die gesamte Oberfläche des in der weiteren Schicht enthaltenen Feuchtigkeitsabsorptionsmittels verstanden. Liegt die weitere Schicht beispielsweise in einer Stärke von 1 mm, einer Breite von 1 cm und einer Länge von 6 cm vor, beträgt deren gesamte äußere Oberfläche 2 x (1 cm x 6 cm + 0,1 cm x 6 cm + 0,1 cm x 1 cm) = 13,4 cm². Angenommen, dass die Größe der Kontaktfläche zwischen der weiteren Schicht und der Schicht in diesem Fall 6 cm² beträgt, beträgt der Anteil dieser Kontaktfläche an der gesamten äußeren Oberfläche von 13,4 cm² 44,8%. Durch die im Verhältnis zum Volumen der weiteren Schicht große Kontaktfläche wird stets eine schnelle Aufnahme von Flüssigkeit aus der Schicht und damit eine lang anhaltende Aufrechterhaltung der Funktion der Schicht ermöglicht.

Die Verbindung der Kunststofffolie mit den äußeren Seitenflächen der Schicht und, sofern vorhanden, der weiteren Schicht und/oder der Membran mit dem Auslassbereich kann mittels eines Klebstoffs bereitgestellt werden. Bei dem Klebstoff kann es sich um einen nicht-thermischen oder einen thermischen Klebstoff handeln. Bei einem thermischen Klebstoff handelt es sich um einen sogenannten Heißkleber, d. **h.** einen Klebstoff, der durch Erwärmen schmilzt oder zumindest oberflächlich anschmilzt und beim Abkühlen eine Klebeverbindung herstellt. Beispielsweise kann dazu auf der Kunststofffolie und/oder der Membran ein sogenanntes Klebevlies aus Ethylenvinylacetat (EVA) aufgebracht sein. Beim Aufbringen der Kunststofffolie und/oder der Membran kann dann gleichzeitig eine Erwärmung des Klebevlieses erfolgen, so dass beim Abkühlen des Klebevlieses eine Klebeverbindung zwischen der Kunststofffolie und/oder der Membran und der äußeren Seitenflächen der Schicht bzw. der weiteren Schicht hergestellt wird.

Weiterhin betrifft die Erfindung einen Stomabeutel mit einem erfindungsgemäßen Filterelement, wobei der Stomabeutel eine außenliegende Kammer aufweist, in welcher das Filterelement enthalten ist. Die Kammer weist dabei mindestens eine erste Öffnung auf, die eine gasdurchlässige Verbindung mit dem inneren des Stomabeutels bereitstellt. Die erste Öffnung kann mit einer gasdurchlässigen, für Wasser in flüssiger Form undurchlässigen weiteren Membran verschlossen sein. Weiterhin weist die Kammer eine nach außen führende zweite Öffnung auf, wobei der Rand der zweiten Öffnung durch Verschweißen mit der Membran des Filterelements oder der Membran und der Kunststofffolie des Filterelements wasserdicht verbunden ist, so dass Gas vom Inneren des Stomabeutels über die erste Öffnung, die Einlassöffnung und die Aktivkohle enthaltende Schicht des Filterelements durch die Membran und die zweite Öffnung nach außen entweichen kann.

Nachfolgend werden Ausgestaltungen der Erfindung anhand der Zeichnungen näher erläutert. Es zeigen:
- Fig. 1: einen Querschnitt durch ein streifenförmiges erfindungsgemäßes Filterelement,
- Fig. 2: das in Fig. 1 gezeigte Filterelement in Aufsicht,
- Fig. 3: einen Querschnitt durch ein weiteres streifenförmiges erfindungsgemäßes Filterelement mit einer weiteren ein Feuchtigkeitsabsorptionsmittel umfassenden Schicht und
- Fig. 4: das in Fig. 3 gezeigte Filterelement in Aufsicht auf die der weiteren Schicht gegenüberliegenden Seite des Filterelements.

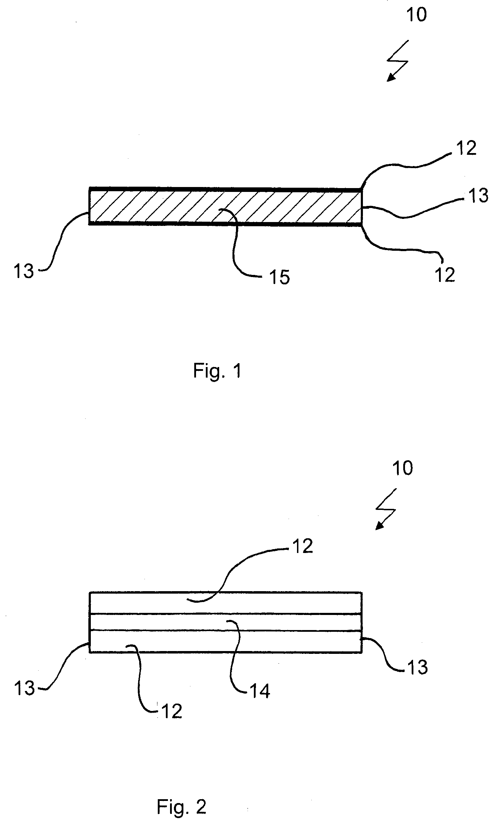

Fig. 1 und 2 zeigen das erfindungsgemäße streifenförmig ausgebildete Filterelement 10 mit einer Aktivkohle enthaltenen Schicht 15 und einer äußeren Umhüllung aus einer Kunststofffolie 12, Einlassöffnungen 13 auf beiden Seiten des Filterelements 10 und einem Auslassbereich, der von einer für Wasser in flüssiger Form undurchlässigen aber für Wasserdampf und Gas durchlässigen Membran 14 bedeckt ist. Beim Einsatz des Filterelements 10 in einem Stomabeutel kann Gas über die beiden Einlassöffnungen 13, die Aktivkohle enthaltende Schicht 15 und den von der Membran 14 bedeckten Auslassbereich durch den Filter strömen. Ein Gasaustritt aus dem Stomabeutel ist bei einem erfindungsgemäßen Stomabeutel nur über eine nach außen führende Öffnung des Stomabeutels, deren Rand mit der Membran 14 oder der Membran 14 und der Kunststofffolie 12 verschweißt und dadurch wasserdicht verbunden ist, möglich.

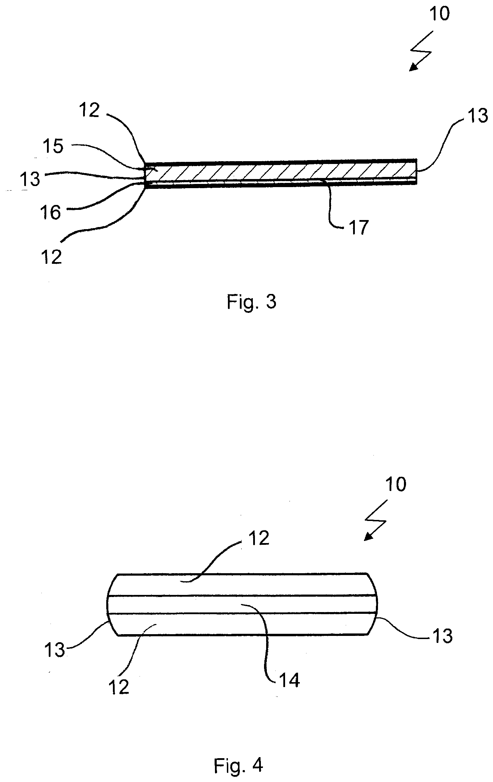

Fig. 3 und 4 zeigen ein weiteres streifenförmig ausgebildetes erfindungsgemäßes Filterelement 10, welches eine äußere Umhüllung aus einer Kunststofffolie 12 aufweist. Das Filterelement 10 weist zwei Einlassöffnungen 13 und eine einen Auslassbereich bedeckende Membran 14 auf. Aus dem in Fig. 3 dargestellten Querschnitt sind die von der Kunststofffolie 12 umgebene Schicht 15 und die weitere Schicht 16 sowie die Kontaktfläche 17 zwischen der weiteren Schicht 16 und der Schicht 15 ersichtlich. Der von der Membran 14 bedeckte Auslassbereich befindet sich dabei auf der Seite der Schicht 15, welche der Kontaktfläche 17 zwischen der Schicht 15 und der weiteren Schicht 16 gegenüberliegt. In der Schicht 15 sind Aktivkohlepartikel enthalten, die in ein aus Polyurethanschaum bestehendes Trägermaterial integriert sind. Die weitere Schicht 16 besteht aus einem Vlies, welches aus einer Trägerfaser und 15 Gew.-% faserförmigem Superabsorber gebildet ist. Der Superabsorber kann von der Firma Evonik Stockhausen GmbH, Bäkerpfad 25, 47805 Krefeld, BTC Speciality Chemical Distribution GmbH, BASF-Gruppe, Maarweg 163/165, 50825 Köln oder Technical Absorbents Ltd., Great Coates, Off Moody Lane, Grimsby, DN31 2SS UK, bezogen werden. Die Trägerfaser kann von der Firma Lenzing AG, A-4860 Lenzing, Werkstraße 2, Österreich bezogen werden.

### Bezugszeichenliste

- 10: Filterelement
- 12: Kunststofffolie
- 13: Einlassöffnung
- 14: Membran
- 15: Schicht
- 16: weitere Schicht
- 17: Kontaktfläche

## Patentansprüche

1. Filterelement (10) zur Reinigung von aus einem Stomabeutel austretenden Gasen, wobei das Filterelement (10) mindestens eine Einlassöffnung (13) zum Eintritt der Gase, einen Auslassbereich zum Austritt der Gase und eine von den Gasen auf ihrem Weg von der Einlassöffnung (13) zum Auslassbereich zu durchströmende Aktivkohle enthaltende Schicht (15) aufweist, wobei das Filterelement (10) in Form eines Streifens vorliegt, **dadurch gekennzeichnet, dass** die Schicht (15) äußere Seitenflächen und Stirnflächen aufweist, wobei ein Teil der äußeren Seitenflächen der Schicht (15) von einer gas- und flüssigkeitsundurchlässigen Kunststofffolie (12) umgeben und mit dieser Kunststofffolie (12) verbunden ist, wobei der nicht von der Kunststofffolie (12) umgebene Teil der äußeren Seitenflächen der Schicht (15) den Auslassbereich bildet, der sich längs des Filterelements (10) erstreckt und der von einer für Wasser in flüssiger Form undurchlässigen aber für Wasserdampf und Gas durchlässigen thermisch oder mittels Ultraschall schweißbaren Membran (14) bedeckt und mit dieser Membran (14) verbunden ist, wobei der Schmelzpunkt der Membran (14) unter 180°C liegt, wobei der überwiegende Teil der von den äußeren Seitenflächen der Schicht (15) gebildeten Fläche von der Kunststofffolie (12) umgeben und damit verbunden ist.

2. Filterelement (10) nach Anspruch 1, wobei die Membran (14) Polycarbonat, Polyvinylidenfluorid (PVDF) oder Acrylcopolymer, insbesondere in Form einer mikroporösen Kapillarporenmembran, umfasst oder daraus besteht oder zu einem Vlies oder einem Gewebe verarbeitete, insbesondere thermisch verschweißte, Fasern, insbesondere Fasern aus Polyethylen, insbesondere Polyethylen hoher Dichte (HDPE), umfasst oder aus solchen Fasern besteht.

3. Filterelement (10) nach einem der vorhergehenden Ansprüche, wobei die Kunststofffolie (12) aus einem thermisch oder mittels Ultraschall mit der Membran (14) verschweißbaren Material, insbesondere Ethylenvinylacetat (EVA), Polyethylen (PE), Polypropylen (PP) oder Ethylenvinylacetat-Polyethylen Coextrudat oder Copolymer (EVA-PE), besteht.

4. Filterelement (10) nach einem der vorhergehenden Ansprüche, wobei das Filterelement (10) in ein Gasauslasssystem eines Stomabeutels integriert ist, wobei der Rand einer nach außen führenden Öffnung des Stomabeutels vollständig so mit der Membran (14) oder der Membran (14) und der Kunststofffolie (12) verschweißt ist, dass kein Wasser in flüssiger Form unter Normaldruck von außen durch die Öffnung in den Stomabeutel eindringen, Gas jedoch durch die Membran (14) aus dem Stomabeutel austreten kann.

5. Filterelement (10) nach einem der vorhergehenden Ansprüche, wobei die Aktivkohle auf oder in einem, insbesondere faserförmigen oder schwammförmigen, Trägermaterial angeordnet oder fixiert ist.

6. Filterelement (10) nach Anspruch 5, wobei das Trägermaterial ein aus einem Kunststoff, insbesondere Polyurethan, gebildeter Schaum ist.

7. Filterelement (10) nach einem der vorhergehenden Ansprüche, wobei neben der Schicht (15) mindestens eine weitere Schicht (16) vorgesehen ist, die ein Feuchtigkeitsabsorptionsmittel umfasst, wobei die weitere Schicht (16) mit der Schicht (15) zur Aufnahme von Feuchtigkeit aus der Schicht (15) in direktem Kontakt steht, wobei die weiter Schicht (16) so bemessen ist, dass sie es auch bei einer Sättigung mit Flüssigkeit noch erlaubt, dass die Gase das Filterelement (10) von der Einlassöffnung (13) zum Auslassbereich durchströmen, wobei die äußeren Seitenflächen der weiteren Schicht (16) zusammen mit dem nicht den Auslassbereich bildenden Teil der äußeren Seitenflächen der Schicht (15) von der Kunststofffolie (12) umgeben und mit ihr verbunden sind.

8. Filterelement (10) nach Anspruch 7, wobei die weitere Schicht (16) einen Superabsorber, insbesondere einen Superabsorber in Form von Fasern, insbesondere von zu einem Vlies verarbeiteten Fasern, umfasst.

9. Filterelement (10) nach Anspruch 7 oder 8, wobei die weitere Schicht (16) mit der Schicht (15) so in Kontakt steht, dass der Anteil einer Kontaktfläche (17) zwischen der weiteren Schicht (16) und der Schicht (15) an der gesamten äußeren Oberfläche der weiteren Schicht (16) einschließlich der Kontaktfläche (17) in trockenem Zustand mindestens 44%, vorzugsweise mindestens 47%, beträgt.

10. Filterelement (10) nach einem der Ansprüche 7 bis 9, wobei eine Verbindung der Kunststofffolie (12) mit den äußeren Seitenflächen der Schicht (15) und der weiteren Schicht (16) und/oder der Membran (14) mit dem Auslassbereich mittels eines Klebstoffs hergestellt ist.

11. Filterelement (10) nach einem der Ansprüche 1 bis 6, wobei eine Verbindung der Kunststofffolie (12) mit den äußeren Seitenflächen der Schicht (15) und/oder der Membran (14) mit dem Auslassbereich mittels eines Klebstoffs hergestellt ist.

12. Stomabeutel, **dadurch gekennzeichnet, dass** dieser ein Filterelement (10) nach einem der vorhergehenden Ansprüche aufweist, wobei der Stomabeutel eine außenliegende Kammer aufweist, in welcher das Filterelement (10) enthalten ist, wobei die Kammer mindestens eine erste Öffnung, die eine gasdurchlässige Verbindung mit dem Inneren des Stomabeutels bereitstellt, und eine nach außen führende zweite Öffnung aufweist, wobei der Rand der zweiten Öffnung durch Verschweißen mit der Membran (14) des Filterelements (10) oder der Membran (14) und der Kunststofffolie (12) des Filterelements (10) wasserdicht verbunden ist, so dass Gas vom Inneren des Stomabeutels über die erste Öffnung, die Einlassöffnung (13) und die Aktivkohle enthaltende Schicht (15) des Filterelements (10) durch die Membran (14) und die zweite Öffnung nach außen entweichen kann.

## Claims

1. Filter element (10) for purifying gases escaping from a stoma bag, the filter element (10) having at least one inlet opening (13) for the entry of the gases, an outlet region for the exit of the gases and a layer (15) containing activated carbon through which the gases are to flow on their way from the inlet opening (13) to the outlet region, wherein the filter element (10) is in the form of a strip, **characterized in that** the layer (15) has outer side surfaces and end surfaces, wherein a part of the outer side surfaces of the layer (15) is surrounded by a gas- and liquid-impermeable plastic film (12) and is connected to this plastic film (12), wherein the part of the outer side surfaces of the layer (15) which is not surrounded by the plastic film (12) forms the outlet region which extends along the filter element (10) and which is covered by a membrane (14) and is connected to this membrane (14), which is impermeable to water in liquid form but permeable to water vapour and gas and which is weldable thermally or by means of or ultrasonic sound, wherein the melting point of the membrane (14) is below 180°C, the major part of the surface formed by the outer side surfaces of the layer (15) being surrounded by the plastic film (12) and bonded thereto.

2. Filter element (10) according to claim 1, wherein the membrane (14) comprises or consists of polycarbonate, polyvinylidene fluoride (PVDF) or acrylic copolymer, in particular in the form of a microporous capillary pore membrane, or comprises or consists of fibers, in particular thermally welded fibers, processed to form a nonwoven or a fabric, which fibers are in particular fibers made of polyethylene, in particular high-density polyethylene (HDPE).

3. Filter element (10) according to one of the preceding claims, wherein the plastic film (12) consists of a material which can be thermally or ultrasonically welded to the membrane (14), which material is in particular ethylene vinyl acetate (EVA), polyethylene (PE), polypropylene (PP) or ethylene vinyl acetate-polyethylene coextrudate or copolymer (EVA-PE).

4. Filter element (10) according to one of the preceding claims, wherein the filter element (10) is integrated into a gas outlet system of a stoma bag, wherein the edge of an outwardly leading opening of the stoma bag is completely welded to the membrane (14) or the membrane (14) and the plastic film (12) in such a way that no water in liquid form under normal pressure can penetrate from the outside through the opening into the stoma bag, but gas can escape from the stoma bag through the membrane (14)

5. Filter element (10) according to one of the preceding claims, wherein the activated carbon is arranged or fixed on or in a carrier material, in particular in the form of fibers or sponge.

6. Filter element (10) according to claim 5, wherein the carrier material is a foam formed from a plastic, in particular polyurethane.

7. Filter element (10) according to one of the preceding claims, wherein in addition to the layer (15) at least one further layer (16) is provided, which comprises a moisture absorbent, wherein the further layer (16) is in direct contact with the layer (15) for absorbing moisture from the layer (15), wherein the further layer (16) is dimensioned in such a way that it still allows the gases to flow through the filter element (10) from the inlet opening (13) to the outlet region even when saturated with liquid, wherein the outer side surfaces of the further layer (16) together with the part of the outer side surfaces of the layer (15) not forming the outlet region are surrounded by and bonded to the plastic film (12).

8. Filter element (10) according to claim 7, wherein the further layer (16) comprises a superabsorber, in particular a superabsorber in the form of fibers, in particular fibers processed into a fleece.

9. Filter element (10) according to claim 7 or 8, wherein the further layer (16) is in contact with the layer (15) such that the proportion of a contact surface (17) between the further layer (16) and the layer (15) on the total outer surface of the further layer (16) including the contact surface (17) in the dry state is at least 44%, preferably at least 47%.

10. Filter element (10) according to one of claims 7 to 9, wherein a connection of the plastic film (12) with the outer side surfaces of the layer (15) and the further layer (16) and/or the membrane (14) with the outlet region is established by means of an adhesive.

11. Filter element (10) according to one of claims 1 to 6, wherein a connection of the plastic film (12) with the outer side surfaces of the layer (15) and/or the membrane (14) with the outlet region is established by means of an adhesive.

12. A stoma bag, **characterized in that** it comprises a filter element (10) according to one of the preceding claims, wherein the stoma bag comprises an external chamber in which the filter element (10) is contained, wherein the chamber comprises at least a first opening which provides a gas-permeable connection with the interior of the stoma bag, and a second opening leading to the outside, wherein the edge of the second opening is connected by welding to the membrane (14) of the filter element (10) or the membrane (14) and the plastic film (12) of the filter element (10) in a watertight manner, so that gas can escape from the interior of the stoma bag via the first opening, the inlet opening (13) and the activated carbon-containing layer (15) of the filter element (10) through the membrane (14) and the second opening to the outside.

## Revendications

1. Élément de filtre (10) pour le nettoyage des gaz sortant d'une poche de stomie, dans lequel l'élément de filtre (10) présente au moins une ouverture d'entrée (13) pour l'entrée des gaz, une région de sortie pour la sortie des gaz et une couche (15) contenant du charbon actif à parcourir par les gaz sur leur trajet de l'ouverture d'entrée (13) à la région de sortie, dans lequel l'élément de filtre (10) est présent sous la forme d'une bande, **caractérisé en ce que** la couche (15) présente des faces latérales extérieures et des faces frontales, dans lequel une partie des faces latérales extérieures de la couche (15) est entourée d'une feuille en plastique (12) imperméable au gaz et au liquide et connectée à cette feuille en plastique (12), dans lequel la partie non entourée de la feuille en plastique (12) des faces latérales extérieures de la couche (15) forme la région de sortie qui s'étend le long de l'élément de filtre (10) et qui est couverte par une membrane (14) pouvant être soudée thermiquement ou au moyen d'ultrasons, imperméable à l'eau sous forme liquide mais perméable à la vapeur d'eau et au gaz et connectée à cette membrane (14), dans lequel le point de fusion de la membrane (14) se situe en dessous de 180 °C, dans lequel la partie prépondérante de la face formée par les faces latérales extérieures de la couche (15) est entourée de et connectée à la feuille en plastique (12).

2. Élément de filtre (10) selon la revendication 1, dans lequel la membrane (14) comprend du polycarbonate, fluorure de polyvinylidène (PVDF) ou copolymère d'acryle, notamment sous forme d'une membrane microporeuse à pores capillaires, ou se compose de ceux-ci ou comprend des fibres transformées en un non tissé ou un tissu, notamment soudées thermiquement, notamment des fibres en polyéthylène, notamment polyéthylène haute densité (HDPE), ou se compose de telles fibres.

3. Élément de filtre (10) selon une des revendications précédentes, dans lequel la feuille en plastique (12) se compose d'un matériau pouvant être soudé thermiquement ou au moyen d'ultrasons à la membrane (14), notamment d'éthylène-acétate de vinyle (EVA), de polyéthylène (PE), de polypropylène (PP) ou d'un co-extrudat ou copolymère d'éthylène-acétate de vinyle-polyéthylène (EVA-PE).

4. Élément de filtre (10) selon une des revendications précédentes, dans lequel l'élément de filtre (10) est intégré dans un système de sortie de gaz d'une poche de stomie, dans lequel le bord d'une ouverture menant à l'extérieur de la poche de stomie est entièrement soudé à la membrane (14) ou la membrane (14) et la feuille en plastique (12) de sorte qu'aucune eau sous forme liquide ne peut pénétrer sous pression normale de l'extérieur à travers l'ouverture dans la poche de stomie, mais que du gaz peut sortir à travers la membrane (14) de la poche de stomie.

5. Élément de filtre (10) selon une des revendications précédentes, dans lequel le charbon actif est disposé ou fixé sur ou dans un matériau de support, notamment fibreux ou spongieux.

6. Élément de filtre (10) selon la revendication 5, dans lequel le matériau de support est une mousse formée d'un plastique, notamment de polyuréthane.

7. Élément de filtre (10) selon une des revendications précédentes, dans lequel au moins une autre couche (16) est prévue outre la couche (15), laquelle comprend un moyen d'absorption d'humidité, dans lequel l'autre couche (16) est en contact direct avec la couche (15) pour l'absorption d'humidité de la couche (15), dans lequel l'autre couche (16) est mesurée de sorte qu'elle permet encore également dans le cas d'une saturation en liquide que les gaz traversent l'élément de filtre (10) de l'ouverture d'entrée (13) à la région de sortie, dans lequel les faces latérales extérieures de l'autre couche (16) sont entourées conjointement à la partie des faces latérales extérieures de la couche (15) ne formant pas la région de sortie de la feuille en plastique (12) et connectées à elle.

8. Élément de filtre (10) selon la revendication 7, dans lequel l'autre couche (16) comprend un super absorbeur, notamment un super absorbeur sous forme de fibres, notamment de fibres transformées en un non tissé.

9. Élément de filtre (10) selon la revendication 7 ou 8, dans lequel l'autre couche (16) est en contact avec la couche (15) de sorte que la proportion d'une face de contact (17) entre l'autre couche (16) et la couche (15) sur la surface extérieure totale de l'autre couche (16), y compris de la face de contact (17), à l'état sec se monte à au moins 44 %, de préférence au moins 47 %.

10. Élément de filtre (10) selon une des revendications 7 à 9, dans lequel une connexion de la feuille en plastique (12) avec les faces latérales extérieures de la couche (15) et de l'autre couche (16) et/ou de la membrane (14) à la région de sortie est fabriquée au moyen d'un adhésif.

11. Élément de filtre (10) selon une des revendications 1 à 6, dans lequel une connexion de la feuille en plastique (12) avec les faces latérales extérieures de la couche (15) et/ou de la membrane (14) à la région de sortie est fabriquée au moyen d'un adhésif.

12. Poche de stomie, **caractérisée en ce que** celle-ci présente un élément de filtre (10) selon une des revendications précédentes, dans laquelle la poche de stomie présente une chambre externe dans laquelle l'élément de filtre (10) est contenu, dans laquelle la chambre présente au moins une première ouverture qui met à disposition une connexion perméable au gaz à l'intérieur de la poche de stomie et une seconde ouverture menant à l'extérieur, dans laquelle le bord de la seconde ouverture est connecté de manière étanche à l'eau par soudage à la membrane (14) de l'élément de filtre (10) ou la membrane (14) et la feuille en plastique (12) de l'élément de filtre (10) de sorte que du gaz peut s'échapper de l'intérieur de la poche de stomie par le biais de la première ouverture, l'ouverture d'entrée (13) et la couche (15) contenant du charbon actif de l'élément de filtre (10) à travers la membrane (14) et la seconde ouverture vers l'extérieur.
